(19) **Europäisches Patentamt**

**European Patent Office**

**Office européen des brevets**

(11) **EP 1 003 454 B1**

(12) **EUROPEAN PATENT SPECIFICATION**

(45) Date of publication and mention
of the grant of the patent:
**18.09.2002 Bulletin 2002/38**

(51) Int Cl.⁷: **A61F 13/15**

(86) International application number:
**PCT/SE98/01238**

(21) Application number: **98932678.0**

(22) Date of filing: **25.06.1998**

(87) International publication number:
**WO 99/001099 (14.01.1999 Gazette 1999/02)**

(54) **LIQUID-PERMEABLE COVER SHEET FOR ABSORBENT ARTICLE**

FLÜSSIGKEITSDURCHLÄSSIGE DECKSCHICHT FÜR ABSORBIERENDE ARTIKEL

FEUILLE DE COUVERTURE PERMEABLE AU LIQUIDE POUR ARTICLE ABSORBANT

(84) Designated Contracting States:
**AT BE CH CY DE DK ES FI FR GB GR IE IT LI LU MC NL PT SE**

(30) Priority: **03.07.1997 SE 9702572**

(43) Date of publication of application:
**31.05.2000 Bulletin 2000/22**

(73) Proprietor: **SCA Hygiene Products AB**
**405 03 Göteborg (SE)**

(72) Inventors:
• **OLOFSSON, Ulla**
**S-430 94 Bohus-Björkö (SE)**
• **CHRISTIANSEN, Karin**
**S-511 63 Skene (SE)**

• **CHIHANI, Thami**
**S-435 32 Mölnlycke (SE)**

(74) Representative: **Romare, Laila Anette**
**Albihns Göteborg AB**
**Box 142**
**401 22 Göteborg (SE)**

(56) References cited:
**EP-A- 0 483 859     US-A- 4 351 784**
**US-A- 4 743 494**

• **PATENT ABSTRACTS OF JAPAN; & JP,A,01 192 871 (KURARAY CO LTD) 2 August 1989.**
• **FELLERS, CHRISTER, NORMAN, BO. PAPPERSTEKNIK, STOCKHOLM: KTH, February 1996, Third Ed., page 119.**

**Description**

TECHNICAL FIELD:

The invention relates to a liquid-permeable cover sheet for an absorbent article such as a diaper, an incontinence protector, a sanitary towel or the like, which cover sheet comprises at least a first material layer.

BACKGROUND:

The liquid-permeable cover sheet is intended to bear against the user's body during use of the absorbent article, which means that this sheet first receives the excreted body fluid. To avoid leakage of the liquid, it is important that the liquid-permeable cover sheet can receive a large amount of liquid during a short period of time. It is also important that the cover sheet withstands repeated wetting, i.e. can retain its liquid permeability when the article has been subjected to a number of wettings.

As liquid-permeable cover sheets, it is customary to use nonwovens and film materials. Such cover sheets are generally made of synthetic materials which are inherently hydrophobic. In order to obtain liquid permeability, it is customary to treat these materials with wetting agents. Plastic films used as liquid-permeable cover sheets must in addition be perforated to become liquid-permeable. However, it is also common for nonwoven materials to be perforated in order to increase the liquid permeability. It is difficult, however, to produce a perforated hydrophobic material in which the risk of leakage is completely eliminated.

Treatment with wetting agents is generally carried out by coating the hydrophobic material with a surface-active substance, such as, for example, a surfactant. In this way, a hydrophilic cover sheet is produced. For a material to be considered liquid-permeable, it is necessary for the surface energy of the liquid in question to be lower than the critical surface energy of the material. This is obtained by the surface-active compounds dissolving in the liquid and reducing the surface energy of the liquid and/or by the surface-active compounds binding to the surface of the material, which results in an increased critical surface energy on the material.

A problem with using cover sheets coated with a surface-active substance is that these cover sheets have a liquid permeability which deteriorates upon repeated wetting. This is due to the fact that the surface-active compounds, which are not anchored to the surface of the cover sheet, dissolve in the body fluid upon the first wetting. On subsequent wetting, the amount of surfactant on the surface of the cover sheet has therefore been substantially reduced, which results in reduced liquid permeability. Another problem when using articles with surfactant-coated cover sheets is that the surface-active compounds can cause skin irritations on account of the fact that they migrate from the cover sheet to the user's skin. A further problem with such cover sheets is that the surface-active compounds also migrate from the cover sheet to the inner absorbent structure during the storage time, the result of which is that the cover sheet has inadequate liquid permeability even upon the first wetting.

European Patent 0,483,859 and US-A-4 351 784 describe a liquid-permeable cover sheet which has been corona-treated in order to withstand repeated wetting. In the corona treatment, the cover sheet is treated with a plasma, which is a gas that has been given so much energy that it has been completely or partially ionized. The contact of the material surface with the high-energy gas results in radicals being formed on the material surface. Different types of functional groups are then introduced to the material surface, such as, for example, oxygen-containing functional groups. Such treatment therefore creates a more stable hydrophilic structure than when the surface is only coated with a surface-active compound, without the compound being chemically bonded to the surface. The material which has been corona-treated is a nonwoven material which consists of polypropylene fibres. However, in the case of this known cover sheet, there is still the problem of the liquid permeability substantially decreasing after a first wetting. A further problem with such cover sheets is that it has been found that the modification is not durable, but weakens on storage.

A similar method for producing a material which has been chemically modified on the surface is by means of plasma treatment. US 4,743,494 and WO 94/28568 describe plasma-treated materials which are suitable for use as, for example, liquid-permeable cover sheets. Plasma treatment gives a more homogeneous mixture than the corona treatment. Otherwise, corona and plasma treatments are more or less of equal value. Plasma treatment thus produces, as in corona treatment, a chemically surface-modified material. However, even in the case of liquid-permeable cover sheets treated in this way, there is still the problem of obtaining a stable hydrophilic surface, i.e. a surface which remains hydrophilic even after repeated wetting.

DESCRIPTION OF THE INVENTION:

The present invention has nonetheless made available a liquid-permeable cover sheet of the type discussed in the introduction, having good liquid permeability even after repeated wetting of the article.

A liquid-permeable cover sheet according to the invention comprises at least a first material layer which is characterized in that the surface of the material layer essentially consists of polyethylene which has been treated with plasma or corona in order to obtain lasting hydrophilicity.

As regards corona-treated and plasma-treated materials, it has been found that different materials show significant differences in the acquired ability to retain the liquid permeability upon repeated wetting. When using corona-treated or plasma-treated materials as liquid-permeable cover sheets for absorbent articles, it has been found that the liquid permeability upon repeated wetting is substantially better for materials with a surface of polyethylene than for materials with a surface of polypropylene. It has also been found that treated polyethylene material has an essentially unaltered liquid permeability after the article has been in storage for some length of time.

According to one advantageous embodiment, the first material layer consists of a nonwoven material. The nonwoven material comprises fibres with a surface of polyethylene. For example, the fibres are two-component fibres consisting of a core of polypropylene or polyester and a surrounding covering of polyethylene.

According to another embodiment, the first material layer is a perforated plastic film which is corona-treated or plasma-treated. Since the treated surface essentially consists of polyethylene, the film has hydrophilic groups which are firmly anchored to the plastic surface. The hydrophilic groups on the film surface facilitate the movement of liquid through the perforations.

A further embodiment has a liquid-permeable cover sheet which consists of a plurality of material layers. The cover sheet preferably consists of two layers. The first material layer is made up in accordance with one of the abovementioned embodiments. The second material layer has a surface made essentially of polypropylene. The second material layer is preferably a nonwoven which is not plasma-treated or corona-treated. The second material layer is expediently situated farthest away from the absorbent body, i.e. nearest the user. Since the second material layer has a thin structure with a grammage of between 6-20 $g/m^2$, the fibre structure presents perforations through which the liquid can pass in order to reach the inner, hydrophilic first material layer. Thus, a hydrophobic and dry surface is obtained nearest the user. Of course, it is also possible for the second material layer to be corona-treated or plasma-treated. When the sheet is used as a liquid-permeable cover for an absorbent article, it is also possible to place the second material layer nearest the absorbent structure. These variants are described in more detail in subsequent illustrative embodiments and examples.

The present invention furthermore concerns an absorbent article such as a diaper, an incontinence protector, a sanitary towel or the like, comprising an absorbent body enclosed between a liquid-impermeable cover sheet and a liquid-permeable cover sheet, which liquid-permeable cover sheet comprises at least a first material layer having a material surface which essentially consists of polyethylene. The material layer has been plasma-treated or corona-treated in order to obtain liquid permeability.

One embodiment concerns an absorbent article which is characterized in that the liquid-permeable cover sheet also comprises a second material layer. According to such an embodiment, the first material layer is situated nearest the absorbent body and the second material layer is situated farthest away from the absorbent body. The second material layer preferably consists of a thin nonwoven of polypropylene which is not corona-treated or plasma-treated. Untreated nonwovens of polypropylene are inherently hydrophobic, which means that the surface nearest the user remains dry even after wetting. Moreover, the second material layer has a thin structure, with a grammage of between 6-20 $g/m^2$, the result of which is that the fibre structure has perforations through which liquid can pass in order to reach the inner, first material layer. It is also possible to perforate the second material layer in order to obtain the desired liquid permeability. In this embodiment, the first material layer, i.e. the inner fibre structure, functions as a drainage material, which has the ability to drain liquid from the upper material layer nearest the user. The first material layer preferably consists of a nonwoven. To obtain a first material layer which rapidly takes up liquid from the material layer situated nearest to the user, the nonwoven material consists, for example, of a bulky, cottonwool-like structure, a perforated plastic film, or the like.

It is also possible for the second material layer to be corona-treated or plasma-treated. Since the material essentially consists of polypropylene fibres, the hydrophilic compounds on the polypropylene fibre surface do not remain so firmly anchored to the surface as the hydrophilic compounds which are created when the treatment is carried out on a surface of polyethylene. This means that, upon wetting, hydrophilic groups dissolve in the body fluid and reduce the surface tension of the liquid, the result of which is that the liquid is more easily absorbed by the inner absorbent structure. Another advantage of this embodiment is that the plasma or corona treatment can be carried out after the first material layer and the second material layer have been laminated together. For liquid to pass through the second material layer, situated nearest to the user, upon subsequent wettings, the second material layer preferably consists of a thin or perforated nonwoven. The second material layer can also consist of a perforated film, a net material, or the like.

According to yet another embodiment, the absorbent article is designed in such a way that the second material layer is situated nearest to the absorbent body and the first material layer is situated farthest away from the absorbent body. Both material layers are preferably corona-treated or plasma-treated in order to increase the liquid permeability. The second material layer, as has already been described, consists of a surface essentially of polypropylene. An advantage of this embodiment is that, when the article is being used, the second material layer does not come into direct contact with the user, which reduces the risk of the hydrophilic groups irritating the user's skin after a first wetting.

BRIEF DESCRIPTION OF THE FIGURES:

The invention will be described in greater detail below with reference to the illustrative embodiments which are shown on the attached drawings.

Fig. 1 shows a liquid-permeable cover sheet according to the invention,
Fig. 2 shows another liquid-permeable cover sheet according to the invention, and
Fig. 3 shows a diaper seen from the side which, during use, is intended to be directed towards the user.

DESCRIPTION OF ILLUSTRATIVE EMBODIMENTS:

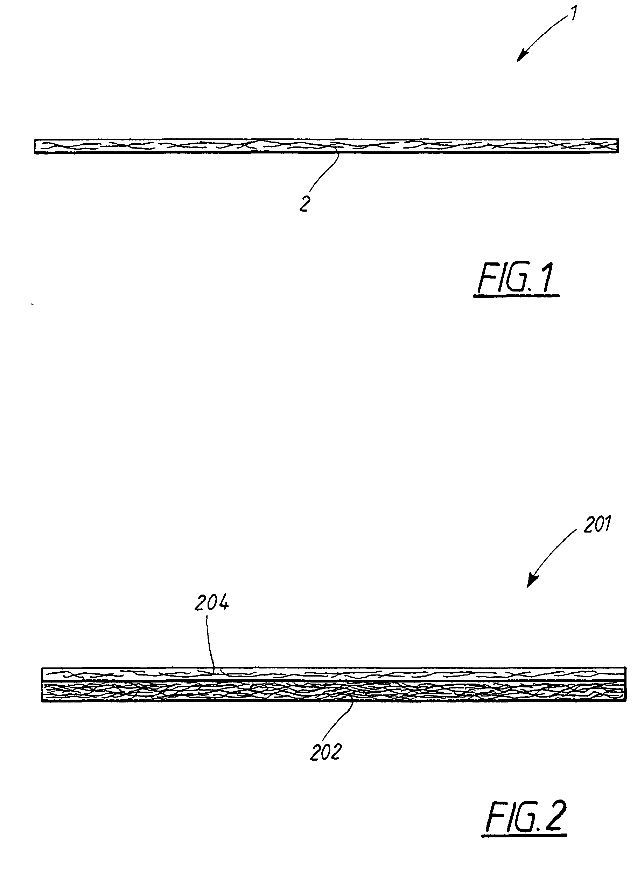

The liquid-permeable cover sheet 1 shown in Figure 1 comprises a material layer 2 which consists of a nonwoven material with fibres having a fibre surface essentially of polyethylene. In order to obtain lasting hydrophilicity, the material layer 2 has been corona-treated or plasma-treated. In the corona or plasma treatment, the material is surface-modified so that hydrophilic groups are chemically bonded to the surface of the material layer 2.

The material 2 is preferably corona-treated or plasma-treated after the fibres have been formed into a nonwoven structure. The treatment is carried out either from only one of the sides of the material layer 2, or from both sides of the material layer 2. It is also possible, however, for the fibres in the material layer 2 to be plasma-treated before the actual formation of the fibres to a nonwoven material.

Examples of different types of polyethylene are LDPE (low-density polyethylene), HDPE (high-density polyethylene) and LLDPE (low linear density poly-ethylene). Examples of fibres that can be used are two-component fibres with a core of polypropylene or polyester and a covering of polyethylene. Of course, the fibres can alternatively consist only of polyethylene, of different types or of the same type. In order to obtain the desired fibre properties, it is also possible to use polyethylene-containing copolymers, for example polyethylene containing a small amount of acrylate, or acetate. The acrylate or acetate component results in the material being more elastic. Moreover, for plasma and corona treatment, it has been found that metallocene-catalyzed polyethylenes are well suited for the purpose.

For a nonwoven spunbonded material to have a high evenness and thus also a high tensile strength, such materials are made up of two different sheets. In order to produce a spunbonded material consisting of two sheet-like structures, material is supplied from two consecutive extruders in the process. Such a method of manufacture makes it possible to produce a spunbonded material consisting of a sheet of polypropylene and a sheet of polyethylene.

Naturally, the material layer 2 is not limited to spunbonded material, but can of course also be of other nonwoven materials such as carded material, or material manufactured in another way. It is also possible for the material layer 2 to consist of a perforated film of polyethylene.

The liquid-permeable cover sheet 201 shown in Figure 2 consists of two material layers. The first material layer 202 is like the material layer 2 described in Figure 1 and thus consists of a corona-treated or plasma-treated nonwoven with fibres having a surface of polyethylene. The second material layer 204 consists of a nonwoven material essentially made up of polypropylene fibres. The second material layer 204 is a thin nonwoven with a grammage of between 6 - 20 g/m$^2$. Of course, the second material layer 204 can also comprise other hydrophobic fibres, or fibre mixtures of two or more different fibre types, such as, for example, different types of polyesters, or nylon. It is also possible for the second material layer 204 to consist of a perforated film of polypropylene.

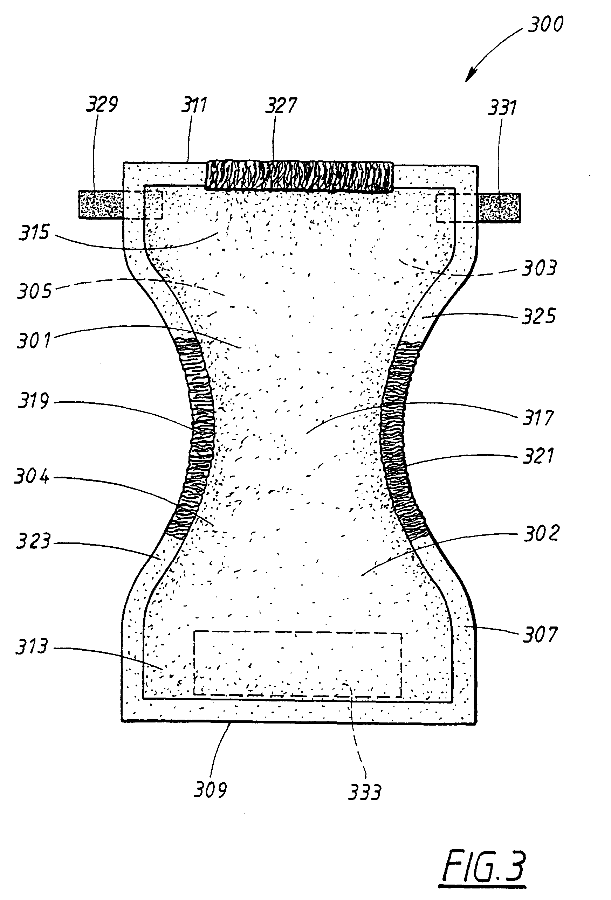

The diaper 300 shown in Figure 3 comprises a liquid-permeable cover sheet 301 in accordance with the invention, a liquid-impermeable cover sheet 303, and an absorbent body 305 enclosed between these. The liquid-impermeable cover sheet 303 can consist of a liquid-impermeable plastic film, a nonwoven sheet which has been coated with a material obstructing liquid, or some other easily pliable material sheet which resists penetration of liquid. It is generally advantageous if the liquid-impermeable cover sheet 303 has a certain breathability, i.e. allows the passage of water vapour. The two cover sheets 301, 303 have a somewhat greater extent in their plane than does the absorbent body 305 and they extend a short distance beyond the edges of the absorbent body 305, all around its periphery. The cover sheets 301, 303 are connected to each other within the protruding areas 307, for example with adhesive or by welding with heat or ultrasound.

The absorbent body 305 is generally made up of one or more sheets of cellulose fibres, for example cellulose fluff pulp. In addition to cellulose fibres, the absorbent body 305 can also contain superabsorbent material, i.e. material in the form of fibres, particles, granules, film or the like, which has the ability to absorb liquid corresponding to several times the actual weight of the superabsorbent material. Superabsorbent material binds the absorbing liquid and thus forms a liquid gel. Moreover, the absorbent body 305 can contain binding agents, shape-stabilizing components, or the like. Further absorbent sheets which improve the absorption properties can also be used, such as different types of liquid-diffusing inlays, or material sheets. The absorbent body 305 can be treated chemically or physically in order to change the absorption properties. It is common, for example, to provide an absorption sheet with compressions in order to control the flow of liquid in the absorbent body 305. Other types of absorption materials can also be used, alone or in combination with cellulose fibres and superabsorbent material. Examples of absorbent materials which can be used are absorbent nonwoven material, foam or the like.

The diaper 301 additionally has two longitudinal side edges 323, 325, a front end edge 309 and a back end edge 311, and it has a front portion 313, a back portion 315 and a narrower crotch portion 317 situated between the front portion 313 and the back portion 315.

Elastic members 319, 321 are also arranged along the side edges 323, 325, at the crotch portion 317 of the diaper. When the diaper is being used, these elastic members 319, 321 serve to keep the diaper sealed tightly around the user's legs. A further elastic member 327 is arranged along the back end edge 311 and is intended to give the diaper 300 a certain stretchability and to serve as a sealing member for the diaper around the user's waist.

A tape flap 329, 331 is arranged on each side edge 323, 325 near the back end edge 311. The tape flaps 329, 331 constitute securing members for the diaper 300 and allow this to be closed together so that it encloses the lower part of a user's trunk like a pair of trousers. The tape flaps 329, 331 cooperate with a receiving area 333 on the liquid-impermeable cover sheet 303 of the diaper 300, at the front portion 313. The receiving area 333 can consist, for example, of a reinforcing material which has been laminated to the liquid-impermeable cover sheet 303. By means of the reinforcement, the diaper 300 can be closed and opened again without the adhesive properties of the tape flaps 329, 331 being impaired, or without the liquid-impermeable cover sheet 303 being torn.

Of course, a number of other types of securing members can be used instead of the described tape flaps 329, 331. Examples of alternative securing members are velcro surfaces, snap-fasten buttons, tie cords, or the like.

The liquid-permeable cover sheet 301 of the diaper is made up of a first material layer 302 and of a second material layer 304. The first material layer 302 is arranged nearest to the absorbent body 305 and the second material layer 304 is arranged nearest to the user when the article is being used. The first material layer 302 is made up in the same way as the material layer 2 according to Figure 1 and thus consists of a nonwoven which is essentially made up of fibres having a surface of polyethylene, or a perforated plastic film with a surface of polyethylene which, in order to obtain lasting liquid permeability, is corona-treated or plasma-treated. The first material layer 302 can also comprise a cotton wool structure essentially consisting of fibres with a surface of polyethylene, which, in order to obtain lasting hydrophilicity, has been corona-treated or plasma-treated. The second material layer 304 is made up in the same way as the material layer 204 shown in Figure 2. The second material layer 304 is thus a nonwoven material essentially made up of polypropylene fibres. The second material layer 304 is preferably not corona-treated or plasma-treated since it lies nearest to the user, but of course it is also possible to treat it with corona or plasma. In order to obtain liquid permeability, it consists of a thin or perforated nonwoven. It is also possible for the second material layer 304 to be made up of a perforated plastic film.

It is also possible for the second material layer 304 to be situated nearest to the absorbent body 305 and for the first material layer 302 to be situated farthest away from the absorbent body 305.

Example 1 - ESCA

In order to examine the chemical composition of the material surface, electron spectroscopic chemical analysis (ESCA) was performed on the following materials:

    1. Plasma-treated nonwoven of polypropylene fibres

        a. Before washing
        b. After washing

    2. Plasma-treated nonwoven of two-component fibres, the fibre cores of polypropylene and the fibre covering of polyethylene

        a. Before washing
        b. After washing

    3. Plasma-treated nonwoven of two-component fibres, the fibre cores of polyester and the fibre covering of polyethylene

        a. Before washing
        b. After washing

    4. Untreated nonwoven of:

        a. polypropylene fibres
        b. two component fibres, the fibre core of polypropylene and the fibre covering of polyethylene
        c. two component fibres, the fibre core of polyester and the fibre covering of polyethylene.

The material is washed by being placed in a container with distilled water. The distilled water is at a temperature of 37°C. The material is left to lie in the water for 15 seconds and is then removed and dried flat.

The material surface is X-rayed on ESCA. The high-energy X-radiation results in electrons being emitted from components of the material surface. The binding energy of the electron is obtained using the following formula:

$$E_b = h\nu - E_k$$

$E_b$ = binding energy of the electron
$E_k$ = kinetic energy of the electron
$h\nu$ = radiation energy

The intensity of the X-radiation is known during the measurement, and the kinetic energy of the electron is obtained by measuring the speed of the electron. Thus, a measurement of the emitted electron's binding energy is obtained, which means that the chemical composition of the surface can be identified.

The following oxygen/carbon ratios (O/C) were found:

| Sample | O/C |
|--------|-------|
| 1a | 0.19 |
| 1b | 0.08 |
| 2a | 0.26 |
| 2b | 0.23 |
| 3a | 0.29 |
| 3b | 0.24 |
| 4a | 0.007 |
| 4b | 0.02 |
| 4c | 0.007 |

The results show that the proportion of oxygen-containing compounds on the material surface is highest for materials 2 and 3, i.e. materials with a fibre covering of polyethylene. This means that the plasma-treated polyethylene surfaces have higher hydrophilicity or wettability than corresponding plasma-treated polypropylene surfaces. In addition, materials 2 and 3 retain a high O/C ratio even after the structure has been washed, which means that polyethylene is superior to polypropylene in terms of retaining wettability after wetting.

The invention must not be seen as being limited to the embodiments described here, and instead a number of other variants and modifications are possible within the scope of the appended patent claims. In addition, all conceivable combinations of the described embodiments are intended to be covered by the invention.

**Claims**

1. A liquid-permeable cover sheet (1, 201, 301) for an absorbent article such as a diaper (300), an incontinence protector, a sanitary towel or the like, which cover sheet comprises at least a first material layer, **characterized in that** the surface of the first material layer (2) essentially consists of polyethylene which has been treated with plasma or corona and in this way has a hydrophilic surface, and that the surface has an oxygen/carbon ratio which is higher than 0.19.

2. A liquid-permeable cover sheet according to Claim 1, **characterized in that** the first material layer (2) consists of a nonwoven material, in which at least the surface of the fibres essentially consists of polyethylene.

3. A liquid-permeable cover sheet according to the preceding claim, **characterized in that** the fibres are two-component fibres consisting of a core of polypropylene and a surrounding covering of polyethylene.

4. A liquid-permeable cover sheet according to Claim 2, **characterized in that** the fibres are two-component fibres consisting of a core of polyester and a surrounding covering of polyethylene.

5. A liquid-permeable cover sheet according to Claim 1, **characterized in that** the first material layer (2) consists of a perforated plastic film.

6. A liquid-permeable cover sheet according to any of the preceding claims and further having a second material layer (204), **characterized in that** the surface of the second material layer (204) essentially consists of polypropylene.

7. A liquid-permeable cover sheet according to Claim 6, **characterized in that** the second material layer (204) is a nonwoven material which essentially is made up of polypropylene fibres.

8. Absorbent article such as a diaper (300), an incontinence protector, a sanitary towel or the like, comprising an absorbent body (305) enclosed between a liquid-impermeable cover sheet (303) and a liquid-permeable cover sheet (301), which liquid-permeable cover sheet (301) comprises at least a first material layer (302), **characterized in that** the surface of the first material layer (302) essentially consists of polyethylene which has been treated with plasma or corona in order to obtain liquid permeability, and that the surface has an oxygen/carbon ratio which is higher than 0.19.

9. Absorbent article according to the preceding claim, **characterized in that** the liquid-permeable cover sheet comprises a second material layer (304) which has a material surface which essentially consists of polypropylene.

10. Absorbent article according to the preceding claim, **characterized in that** the first material layer (302) is situated nearest the absorbent body (305) and **in that** the second material layer (304) is situated farthest from the absorbent body (305).

11. Absorbent article according to Claim 9, **character-**

**ized in that** the second material layer (304) is situated nearest the absorbent body (305) and **in that** the first material layer (302) is situated farthest from the absorbent body (305).

## Patentansprüche

1.  Flüssigkeitsdurchlässige Decklage (1, 201, 301) für einen absorbierenden Gegenstand, wie eine Windel (300), einen Inkontinenzschutz, eine Damenbinde oder dgl., wobei die Decklage mindestens eine erste Materialschicht umfaßt, **dadurch gekennzeichnet, daß** die Oberfläche der ersten Materialschicht (2) im wesentlichen aus Polyethylen besteht, das Plasma- oder Korona-behandelt wurde und auf diese Weise eine hydrophile Oberfläche besitzt, und daß die Oberfläche ein Sauerstoff/Kohlenstoff-Verhältnis von mehr als 0,19 aufweist.

2.  Flüssigkeitsdurchlässige Decklage gemäß Anspruch 1, **dadurch gekennzeichnet, daß** die erste Materialschicht (2) aus einem Nonwoven-Material besteht, in welchem zumindest die Oberfläche der Fasern im wesentlichen aus Polyethylen besteht.

3.  Flüssigkeitsdurchlässige Decklage gemäß dem vorangehenden Anspruch, **dadurch gekennzeichnet daß** die Fasern Zweikomponentenfasern sind, die aus einen Polypropylen-Kern und einer diesen umgebenden Polyethylen-Umhüllung bestehen.

4.  Flüssigkeitsdurchlässige Decklage gemäß Anspruch 2, **dadurch gekennzeichnet, daß** die Fasern Zweikomponentenfasern sind, die aus einem PolyesterKern und einer diesen umgebenden Polyethylen-Umhüllung bestehen.

5.  Flüssigkeitsdurchlässige Decklage gemäß Anspruch 1, **dadurch gekennzeichnet, daß** die erste Materialschicht (2) aus einer perforierten Kunststoffolie besteht.

6.  Flüssigkeitsdurchlässige Decklage gemäß einem der vorangehenden Ansprüche, welche ferner eine zweite Materialschicht (204) aufweist, **dadurch gekennzeichnet, daß** die Oberfläche der zweiten Materialschicht (204) im wesentlichen aus Polypropylen besteht.

7.  Flüssigkeitsdurchlässige Decklage gemäß Anspruch 6, **dadurch gekennzeichnet, daß** die zweite Materialschicht (204) ein Nonwoven-Material ist, das im wesentlichen aus Polypropylen-Fasern besteht.

8.  Absorbierender Gegenstand, wie eine Windel (300), ein Inkontinenzschutz, eine Damenbinde oder dgl., welcher einen absorbierenden Körper (305) umfaßt, der zwischen einer flüssigkeitsundurchlässigen Decklage (303) und einer flüssigkeitsdurchlässigen Decklage (301) eingeschlossen ist, wobei die flüssigkeitsdurchlässige Decklage (301) mindestens eine erste Materialschicht (302) umfaßt, **dadurch gekennzeichnet, daß** die Oberfläche der ersten Materialschicht (302) im wesentlichen aus Polyethylen besteht, das Plasma- oder Korona-behandelt wurde, um die Flüssigkeitsdurchlässigkeit zu erhalten, und daß die Oberfläche ein Sauerstoff/Kohlenstoff-Verhältnis von mehr als 0,19 aufweist.

9.  Absorbierender Gegenstand gemäß dem vorangehendem Anspruch, **dadurch gekennzeichnet, daß** die flüssigkeitsdurchlässige Decklage eine zweite Materialschicht (304) umfaßt, die eine Materialoberfläche aufweist, die im wesentlichen aus Polypropylen besteht.

10. Absorbierender Gegenstand gemäß einem der vorangehenden Ansprüche, **dadurch gekennzeichnet, daß** die erste Materialschicht (302) dem absorbierenden Körper (305) am nächsten liegt und daß die zweite Materialschicht (304) am weitesten vom absorbierenden Körper (305) gelegen ist.

11. Absorbierender Gegenstand gemäß Anspruch 9, **dadurch gekennzeichnet, daß** die zweite Materialschicht (304) dem absorbierenden Körper (305) am nächsten gelegen ist und daß die erste Materialschicht (302) am weitesten vom absorbierenden Körper (305) gelegen ist.

## Revendications

1.  Feuille de couverture (1, 201, 301) perméable aux liquides pour un article absorbant tel qu'une couche (300), une protection pour incontinents, une serviette hygiénique ou des articles similaires, laquelle feuille de couverture comprend au moins une première couche, **caractérisée par le fait que** la surface de la première couche (2) est constituée essentiellement de polyéthylène qui a subi un traitement plasma ou corona et auquel on a conféré ainsi une surface hydrophile, et que la surface a un rapport oxygène/carbone supérieur à 0,19.

2.  Feuille de couverture perméable aux liquides selon la revendication 1, **caractérisée par le fait que** la première couche (2) est constituée d'un matériau non tissé de fibres dont au moins la surface est essentiellement constituée de polyéthylène.

3.  Feuille de couverture perméable aux liquides selon l'une des revendications précédentes, **caractéri-**

**sée par le fait que** les fibres sont des fibres bicomposants constituées d'une âme en polypropylène et d'une enveloppe en polyéthylène.

4. Feuille de couverture perméable aux liquides selon la revendication 2, **caractérisée par le fait que** les fibres sont des fibres bicomposants constituées d'une âme en polyester et d'une enveloppe en polyéthylène.

5. Feuille de couverture perméable aux liquides selon la revendication 1, **caractérisée par le fait que** la première couche de matériau (2) est un film perforé en matière plastique.

6. Feuille de couverture perméable aux liquides selon l'une quelconque des revendications précédentes, comprenant en outre une seconde couche (204), **caractérisée par le fait que** la surface de la seconde couche (204) est constituée essentiellement de polypropylène.

7. Feuille de couverture perméable aux liquides selon la revendication 6, **caractérisée par le fait que** la seconde couche (204) est un matériau non tissé constitué essentiellement de fibres en polypropylène.

8. Article absorbant tel qu'une couche (300), une protection pour incontinents, une serviette hygiénique ou un article similaire, comprenant un corps absorbant (305) pris entre une feuille de couverture (303) imperméable aux liquides et une feuille de couverture (301) perméable aux liquides, laquelle feuille de couverture perméable aux liquides (301) comprend au moins une première couche (302), **caractérisé par le fait que** la surface de la première couche (302) est constituée essentiellement de polyéthylène qui a subi un traitement plasma ou corona lui conférant sa perméabilité aux liquides, et que la surface a un rapport oxygène/carbone supérieur à 0,19.

9. Article absorbant selon la revendication précédente, **caractérisé par le fait que** la feuille de couverture perméable aux liquides comprend une seconde couche (304) qui a une surface essentiellement constituée de polypropylène.

10. Article absorbant selon la revendication précédente, **caractérisé par le fait que** la première couche (302) est celle située le plus proche du corps absorbant (305) et que la seconde couche (304) est celle située le plus loin du corps absorbant (305).

11. Article absorbant selon la revendication 9, **caractérisé par le fait que** la seconde couche (304) est celle située le plus proche du corps absorbant (305) et que la première couche (302) est celle située le plus loin du corps absorbant (305).

_1_

_2_

<u>_FIG.1_</u>

_201_

_204_

_202_

<u>_FIG.2_</u>

_FIG.3_